# EUROPÄISCHE PATENTSCHRIFT

(11) **EP 0 712 636 B1**
(45) Veröffentlichungstag und Bekanntmachung des Hinweises auf die Patenterteilung: **11.07.2001**
(21) Anmeldenummer: 95116799.8
(22) Anmeldetag: 25.10.1995
(51) Int. Cl.: A61M 15/00

(54) **Tragbares Inhalator-Kompressorgerät**
Portable inhaler with compressor
Inhalateur portable à compresseur

(30) Priorität: 15.11.1994 DE 9418334 U
(43) Veröffentlichungstag der Anmeldung: 22.05.1996
(73) Patentinhaber: PARI GmbH Spezialisten für effektive Inhalation, 82319 Starnberg (DE)
(72) Erfinder: Brugger, Stephan, 82301 Starnberg (DE)
(74) Vertreter: Zangs, Rainer E., Dipl.-Ing.

(56) Entgegenhaltungen:
- DE-A- 2 449 816
- DE-A- 3 043 325
- DE-A- 4 306 458
- US-A- 5 022 587

## Beschreibung

Gegenstand der Erfindung ist ein am Körper tragbares Inhalator-Kompressorgerät, d.h. ein kompaktes und tragbares Gerät, welches einen Kompressor, einen Luftfilter und eine Energieversorgung enthält, und welches zusammen mit einem Aerosolvernebler die ortsungebundene Durchführung einer Medikamentenverabreichung durch Inhalation ermöglicht.

Die Verabreichung von Medikamenten in Form eines einzuatmenden Aerosols ist eine weitverbreitete Therapieform. Hierbei gilt es einen Wirkstoff so aufzubereiten, daß eine Suspension von Tröpfchen oder Teilchen in Luft entsteht, wobei der mittlere Durchmesser dieser Tröpfchen oder Teilchen gezielt einstellbar sein sollte, da die Größe angibt wo im Körper der Wirkstoff beim Inhalieren vornehmlich deponiert wird; größere Teilchen werden hauptsächlich im Mund- und Rachenraum abgeschieden, kleinere dringen bis in die Bronchien und Lungen vor. Die Tröpfchen können direkt aus der zu verabreichenden flüssigen Wirkstofflösung bestehen, oder aber auch aus in Flüssigkeit suspendierten festen Wirkstoffpartikeln. Schließlich ist es auch möglich einen pulverförmigen Feststoff direkt mit Luft zu vermischen.

Ein bekanntes Prinzip der Aerosolerzeugung ist die Zuführung von von einem Kompressor verdichteter Luft zu einer Dispergiervorrichtung, beispielsweise einer Düse, in der die unter einem vorbestimmten Druck stehende Luft mit der den Wirkstoff enthaltenden Flüssigkeit vermischt wird. Ein Solches Gerät ist beispielsweise aus DE-A-3 043 325 und US-A-5 022 587 bekannt.

Die bekannten Kompressor-Inhaltionsgeräte haben jedoch den Nachteil, daß der Kompressor voluminös ist und die Abmessungen des Gesamtgerätes bestimmt. Solche bekannten Geräte sind kleinstenfalls Tisch- bzw. Koffergeräte, die zwar transportabel sind, jedoch zu groß sind um sie ständig bei sich zu führen oder an beliebigen Orten einzusetzen.

Im Rahmen vieler Therapien ist es aber notwendig, daß ein Patient in kurzen Intervallen, d.h. mehrmals am Tag inhaliert. Dies kann seine Ursache darin haben, daß eine ständige Medikamentzufuhr nötig ist zur Beherrschung von Symptomen oder auch weil eine bestimmte Tagesgesamtdosis nicht auf einmal verabreicht werden kann. In diesem Fall ist es wünschenswert, daß der Patient ein Inhalationsgerät mit sich führen kann, so daß seine Mobilität nicht eingeschränkt wird und er damit seinem gewohnten Lebensrhythmus folgen kann.

Zu diesem Zweck gibt es seit langem Tascheninhalatoren mit Handpumpen oder mit Druckpatronen. Diese erlauben jedoch nur eine sehr grobe Dosierung und haben nur einen geringen Wirkungsgrad was das Verhältnis von eingesetzter zu verwertbarer Medikamentmenge angeht, so daß solche Inhalatoren für eine dosisgenaue Therapie ungeeignet sind. Ebenfalls bekannt ist eine kompakte, elektrisch betriebene Vorrichtung, die einen Ultraschallvernebler aufweist. Solche Ultraschallvernebler haben jedoch Nachteile gegenüber kompressorbetriebenen Inhalationsgeräten was die Einsatzbreite mit verschiedenen Medikamentenformulierungen sowie die Steuerbarkeit der Aerosolapplikation angeht.

Es ist die Aufgabe der vorliegenden Erfindung, das Kompressorgerät für ein tragbares, kompressorbetriebenes Inhalationsgerät zu schaffen welches von einem Patienten problemlos und bequem ständig bei sich geführt und am Körper getragen werden kann und dabei jederzeit einsetzbar ist. Das Gehäuse des Kompressorgeräts sollte dabei in Form und Größe an das Verneblergefäß angepaßt sein.

Die Lösung dieser Aufgabe geschieht mit einer Vorrichtung gemäß des Wortlauts des ersten Schutzanspruches.

Die Erfindung wird nun anhand von Ausführungsbeispielen und unter Bezugnahme auf die begleitenden Figuren erläutert, wobei die Zeichnungen folgendes zeigen:
- Fig. 1: zeigt eine Perspektivansicht des erfindungsgemäßen Inhalator-Kompressorgeräts;
- Fig. 2: zeigt das Kompressorgehäuse und den herausnehmbaren Energieversorgungsteil;
- Fig. 3: zeigt die Anordnung des Kompressors, des Luftfilters und des elektrischen Einschalters auf dem Rückenelement des Gehäuses;
- Fig. 4: zeigt den Zusammenbau der Teile, die zur Anordnung von Fig. 3 führen;
- Fig. 5: zeigt den Aufbau des Kompressors;
- Fig. 6: zeigt den Aufbau des Zuluftfilters.

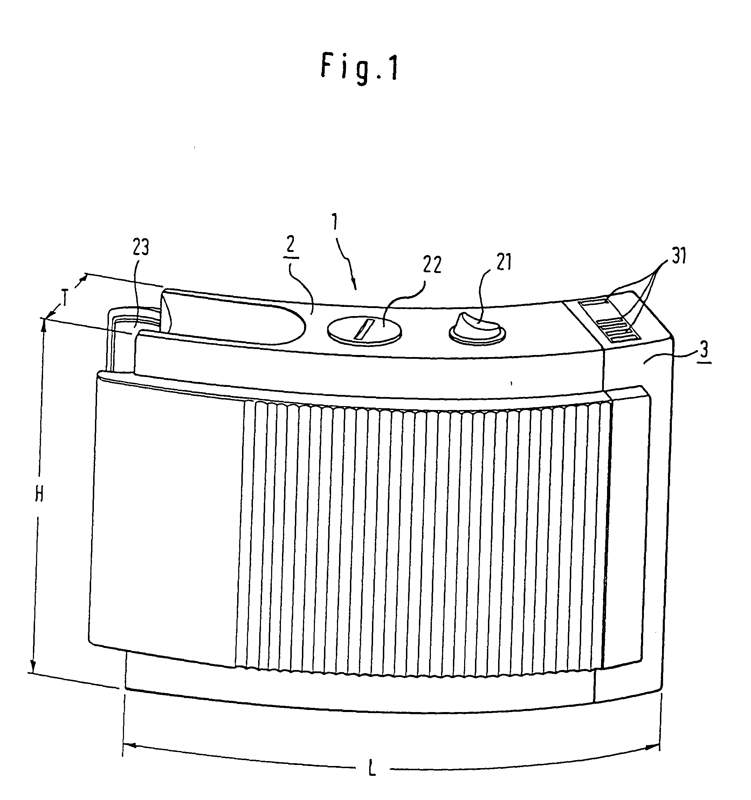

In Figur 1 ist eine Perspektivansicht des Kompressorgerätes 1 dargestellt. Es besteht aus dem Kompressorgehäuse 2 und dem Energieversorgungsteil 3. Das Kompressorgehäuse umfaßt eine Schaltertaste 21 und eine Luftfilteraufnahme 22. An dem dem Energieversorgungsteil 3 gegenüberliegenden Ende des Geräts ist eine Halterung 23 zur Aufnahme eines Verneblergefäßes (nicht abgebildet) vorgesehen. Dies ermöglicht den Betrieb des Kompressors zusammen mit verschiedenen Verneblergefäßen und gibt der Erfindung damit eine hohe Flexibilität in Bezug auf den individuellen Einsatz bei bestimmten Patienten bzw. bei speziellen Therapien.

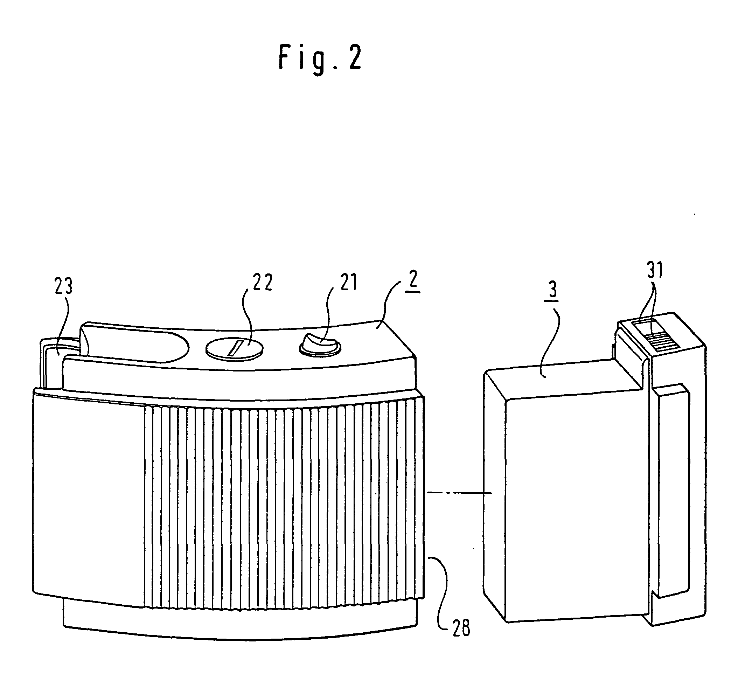

Figur 2 zeigt, daß das Energieversorgungsteil 3 vorzugsweise aus einer Aussparung 28 im Kompressorgehäuse 2 herausnehmbar ist. Dieses Energieversorgungsteil 3 ist gewöhnlich ein einfacher Akkumulator, der an einem externen Ladegerät aufgeladen werden kann und über Anzeigeelemente 31 Auskunft über seinen Betriebs- und Ladezustand gibt. Das Energieversorgungsteil 3 kann jedoch auch ein Netzadapter sein um das Kompressorgerät 1 an eine Netzversorgung anzuschließen wenn eine solche zur Verfügung steht. Hierunter ist nicht nur das öffentliche Stromversorgungsnetz ("Steckdose") zu verstehen, sondern alle möglichen externen Energiequellen, wie z.B. Zigarettenanzünder in Kraftfahrzeugen. Schließlich ist auch ein kombiniertes Energieversorgungsteil 3 denkbar, in welchem Akkumulator und Adapter in einem einzigen Einsteckelement vereinigt sind. Hierbei könnte der Energieversorgungsteil dann so aufgebaut sein, daß bei Betrieb des Gerätes mit einer Energieversorgung aus einem externen Netz gleichzeitig der Akkumulator aufgeladen wird.

Durch diese flexible Energieversorgung ist gewährleistet, daß das Kompressorgerät 1 stets einsatzbereit ist und gleichzeitig aber auch immer mit der gerade zur Verfügung stehenden, den Akkumulator schonenden externen Energiequelle betrieben werden kann.

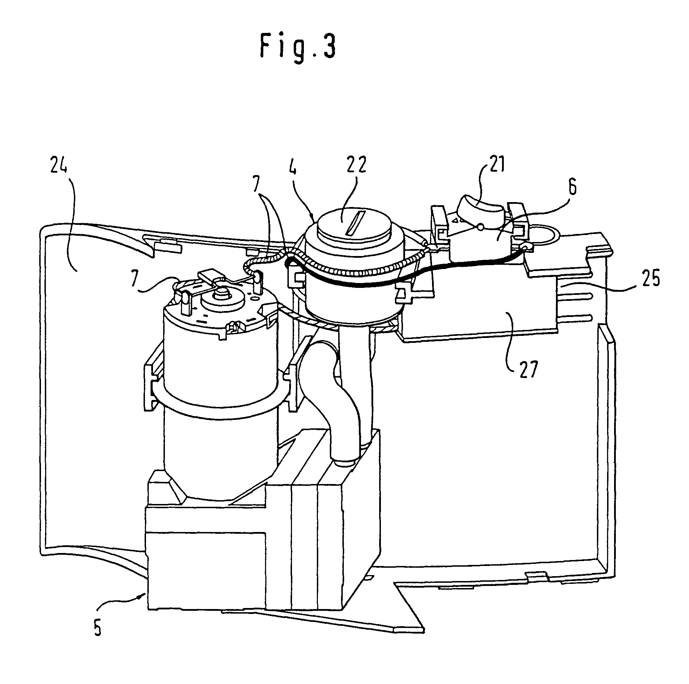

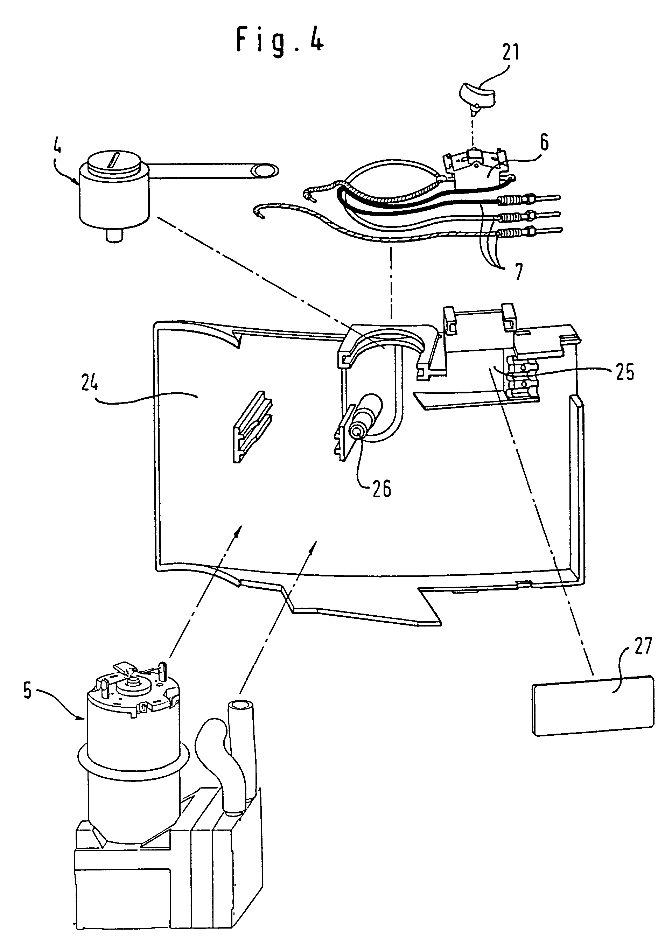

In den Figuren 3 und 4 ist der innere Aufbau des Kompressorgehäuses 2 dargestellt. Man erkennt einen Ansaugluftfilter 4, einen Kompressor 5, einen elektrischen Schalter 6, sowie elektrische Verbindungselemente 7, die alle zusammen auf dem Rückensegment 24 des Kompressorgehäuses 2 angeordnet sind. An einem mit einer Platte 27 abgeschlossenen Energieaufnahmeteil 25 wird eine elektrische Verbindung zum Energieversorgungsteil 3 geschaffen, so daß die elektrischen Verbindungselemente 7 den Kompressor 5 über den Schalter 6 mit dem Energieversorgungsteil 3 verbinden wenn das Energieversorgungsteil 3 eingesetzt ist. Im einfachsten Fall ist der Schalter 6 ein einfacher Ein/Aus-Schalter, es kann aber auch ein mehrstufiger Schalter vorgesehen werden zum mehrstufigen Kompressorbetrieb.

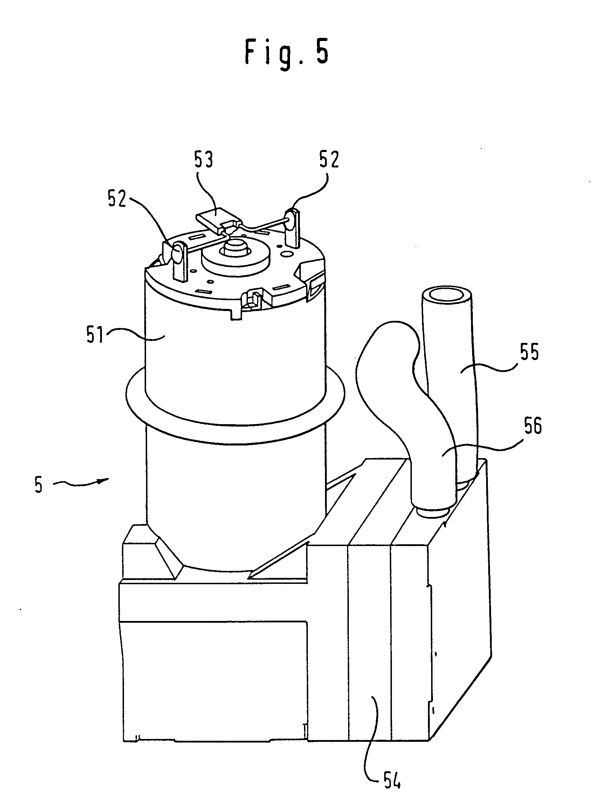

In Figur 5 ist der Kompressor 5 dargestellt. Dieser umfaßt einen Elektromotor 51, der über Anschlüsse 52 mit Energie versorgt wird, wobei diese Anschlüsse 52 vorzugsweise über einen Kondensator 53 verbunden sind. Ferner umfaßt der Kompressor 5 einen eigentlichen Kompressorteil 54, welcher die Kompressionsmechanik enthält, sowie einen Zuluftschlauch 55 bzw. ein Zuluftrohr 55 und einen Ausgangsschlauch 56 bzw. ein Ausgangsrohr 56. Das Zuluftelement 55 ist mit dem Zuluftfilter 4 verbunden, während das Ausgangselement 56 zur Abführung der verdichteten Luft mit dem Ausgangsstutzen 26 verbunden ist. Der Ausgangsstutzen 26 kann an seinem dem Ausgangselement 56 abgewandten Ende (nicht abgebildet) über eine Schnittstelle (nicht abgebildet) verfügen, über die ein Vernebler an den Kompressor angeschlossen werden kann, die Schnittstelle kann jedoch auch an der Verbindung zum Vernebler (nicht abgebildet) vorgesehen sein, so daß das dem Ausgangselement 56 abgewandte Ende eine einfache Öffnung ist.

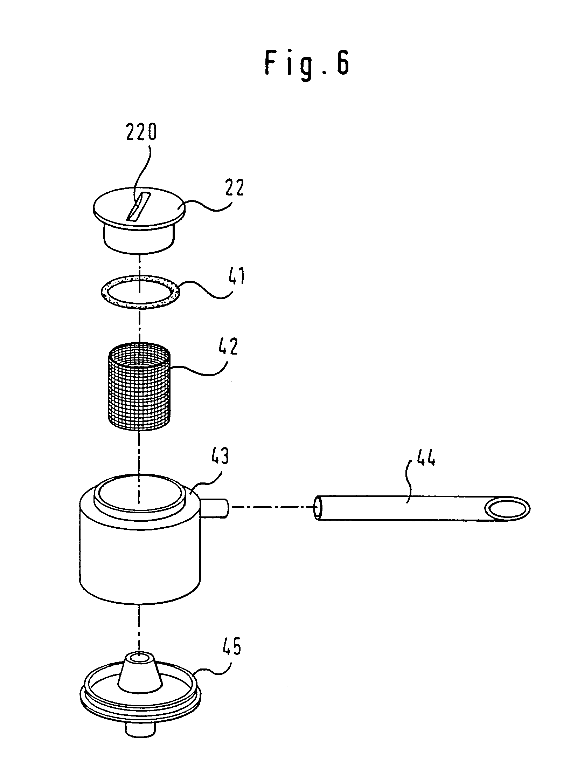

Figur 6 zeigt den Aufbau des Zuluftfilters 4. Es umfaßt die Filteraufnahme 22, einen O-Ring 41, das eigentliche Filterelement 42, das Filtergehäuse 43, einen Ansaugschlauch 44 sowie den Filterboden 45. Diese werden wie in Fig. 6 angedeutet zusammengesetzt, und wie in den Figuren 3 und 4 gezeigt mit dem Kompressor 5 und dem Rückensegment 24 verbunden. Die Filteraufnahme 22 umfaßt einen Drehverschluß zum Fixieren des Filterelements 42 im Filtergehäuse 43. In die Kerbe 220 im Deckel der Filteraufnahme 22 paßt z.B. eine Münze, so daß jeder Benutzer des Geräts das Filterelement 42 durch Aufdrehen der Filteraufnahme 22 bei Bedarf wechseln kann.

Im einfachsten Fall ist das Filterelement 42 ein einfacher Staubfilter um zu verhindern, daß der Kompressor verstopft, es ist aber auch denkbar, speziellere Filter einzusetzen, die zusätzlich bestimmte Stoffe bevorzugt filtern, wie z.B. Feuchtigkeit. Ebenfalls möglich ist das Vorsehen eines Sensors (nicht abgebildet) zur Bestimmung des Beladungszustandes, wobei ein notwendiger Wechsel des Filterelements 42 dann über ein geeignetes Anzeigeinstrument, z.B. eine Kontrolleuchte, angezeigt werden kann.

Das Gerät ist vorzugsweise so zu dimensionieren, daß Tiefe (T in Fig. 1) und Höhe (H in Fig. 1) des Kompressorgehäuses 2 und des Energieversorgungsteils 3 der Tiefe und Höhe des Verneblergefäßes (nicht abgebildet) entsprechen, so daß ein kompaktes und leicht zu handhabendes Inhaliergerät entsteht. Dies ist bei dem in den Zeichnungen gezeigten Ausführungsbeispiel dadurch erreicht, daß der Kompressor 5 und der Zuluftfilter 4 übereinander im mittleren Bereich des Gehäuses angeordnet sind, so daß auf der einen Seite Raum gelassen wird für die Verneblerhalterung 24 und auf der anderen für das Energieversorgungsteil 3.

In einer weiteren bevorzugten Ausführungsform ist die Form des Kompressorgehäuses in seiner Längsausdehnung (L in Fig. 1) leicht gekrümmt, wie in Figur 1 angedeutet. Dies erleichert das Tragen des Geräts am Körper, z.B. an der Hüfte mit Hilfe einer Gürteltasche oder eines Gürtelhalters. Ebenso ist das Gehäuse vorzugsweise mit einer gerippten Oberfläche zu versehen, um eine sichere Handhabung des Geräts zu gewährleisten, das heißt, daß es nicht leicht aus der Hand gleitet.

## Patentansprüche

1. Am Körper tragbares Inhalator-Kompressorgerät (1) umfassend:
- ein Kompressorgehäuse (2), in welchem ein elektrisch angetriebener Kompressor (5) und ein Zuluftfilter (4) zur Filterung der dem Kompressor (5) zugeführten Luft angeordnet sind und welches mit einer Halterung (23) zur Aufnahme eines Verneblergefäßes, dessen Breite im wesentlichen die Breite des Kompressorgehäuses bestimmt, ausgestattet ist, und
- ein Energieversorgungsteil (3), welches zur Versorgung des Kompressors mit elektrischer Energie in eine Aussparung (28) im Kompressorgehäuse einführbar ist.

2. Tragbares Inhalator-Kompressorgerät nach Anspruch 1, dadurch gekennzeichnet, daß der Energieversorgungsteil (3) einen Akkumulator umfaßt.

3. Tragbares Inhalator-Kompressorgerät nach Anspruch 1 oder 2, dadurch gekennzeichnet, daß der Energieversorgungsteil (3) einen Netzadapter zur Versorgung des Gerätes mit Energie aus einem externen Netz umfaßt.

4. Tragbares Inhalator-Kompressorgerät nach einem der Ansprüche 1 bis 3, dadurch gekennzeichnet, daß die Form des Kompressorgehäuses (2) und des Energieversorgungsteils (3) so gewählt sind, daß die Form des Inhalator-Kompressorgeräts (1) in seiner Längsausdehnung (L) gekrümmt ist.

5. Tragbares Inhalator-Kompressorgerät nach einem der Ansprüche 1 bis 4,
dadurch **gekennzeichnet,** daß die Halterung (23) an einem schmalseitigen Ende des Kompressorgehäuses (2) und die Aussparung (28) an dem gegenüberliegenden, schmalseitigen Ende des Kompressorgehäuses (2) angeordnet ist.

6. Tragbares Inhalator-Kompressorgerät nach Anspruch 5,
dadurch **gekennzeichnet,** daß
die Länge des Verneblergehäuses im wesentlichen die Höhe des Kompressorgehäuses (2) bestimmt.

## Claims

1. Inhaler-compressor device (1), portable on the body, comprising:
- a compressor housing (2), in which an electrically driven compressor (5) and an air inlet filter (4) for filtering the air delivered to the compressor (5) are arranged and which is fitted with a holder (23) to accommodate a nebuliser container, the width of which substantially determines the width of the compressor housing, and
- a power supply part (3), which can be inserted into an opening (28) in the compressor housing for supplying the compressor with electrical power.

2. Portable inhaler-compressor device according to Claim 1,
characterised in that the energy supply part (3) comprises an accumulator.

3. Portable inhaler-compressor device according to Claim 1 or 2, characterised in that the energy supply part (3) comprises a network adapter for supplying the device with power from an external network.

4. Portable inhaler-compressor device according to one of Claims 1 to 3, characterised in that the shape of the compressor housing (2) and the energy supply part (3) are chosen in such a way that the shape of the inhaler-compressor device (1) is curved in its longitudinal extension (L).

5. Portable inhaler-compressor device according to one of Claims 1 to 4, characterised in that the holder (23) is arranged on one narrow-sided end of the compressor housing (2) and the opening (28) on the opposite narrow-sided end of the compressor housing (2).

6. Portable inhaler-compressor device according to Claim 5, characterised in that the length of the nebuliser housing substantially determines the height of the compressor housing (2).

## Revendications

1. Inhalateur-compresseur (1) portable sur le corps, comprenant:
un boîtier de compresseur (2) dans lequel sont disposés un compresseur (5) entraîné électriquement, et un filtre (4) à air entrant, pour filtrer l'air amené au compresseur (5), et qui est équipé d'un support (23) pour la réception d'un récipient de brumisation dont la largeur définit essentiellement la largeur du boîtier du compresseur, et
une partie (3) d'alimentation en énergie qui peut être insérée dans une découpe (28) ménagée dans le boîtier du compresseur, pour alimenter le compresseur en énergie électrique.

2. Inhalateur-compresseur portable selon la revendication 1, caractérisé en ce que la partie (3) d'alimentation en énergie comprend un accumulateur.

3. Inhalateur-compresseur portable selon la revendication 1 ou 2, caractérisé en ce que la partie (3) d'alimentation en énergie comprend un adaptateur réseau pour l'alimentation de l'appareil en énergie provenant d'un réseau externe.

4. Inhalateur-compresseur portable selon l'une des revendications 1 à 3, caractérisé en ce que la forme du boîtier (2) du compresseur et celle de la partie (3) d'alimentation en énergie sont sélectionnées de telle sorte que la forme de l'inhalateur-compresseur (1) est courbe dans son étendue longitudinale (L).

5. Inhalateur-compresseur portable selon l'une des revendications 1 à 4, caractérisé en ce que le support (23) est disposé à une extrémité du côté étroit du boîtier (2) du compresseur, et la découpe (28) est disposée à l'extrémité du côté étroit opposé du boîtier (2) du compresseur.

6. Inhalateur-compresseur portable selon la revendication 5, caractérisé en ce que la longueur du boîtier du brumisateur définit essentiellement la hauteur du boîtier (2) du compresseur.
